(19) Europäisches Patentamt
European Patent Office
Office européen des brevets

(11) Publication number: **0 381 713 B1**

# (12) EUROPEAN PATENT SPECIFICATION

(45) Date of publication of patent specification: **19.01.94**

(51) Int. Cl.[5]: **A61K 49/02**, C07C 209/62, A61K 39/395, C07C 323/41

(21) Application number: **89907407.4**

(22) Date of filing: **28.04.89**

(86) International application number: **PCT/US89/01827**

(87) International publication number: **WO 89/10759 (16.11.89 89/27)**

## (54) DIAMINEDITHIOL CHELATING AGENTS FOR RADIOPHARMACEUTICALS.

(30) Priority: **29.04.88 DE 8820084 U**

(43) Date of publication of application: **16.08.90 Bulletin 90/33**

(45) Publication of the grant of the patent: **19.01.94 Bulletin 94/03**

(84) Designated Contracting States: **AT BE CH DE FR GB IT LI NL SE**

(56) References cited:
**EP-A- 200 492**
**US-A- 4 638 051**
**US-A- 4 670 545**
**US-A- 4 673 562**
**US-A- 4 751 286**

**NUCLEAR MEDICINE, vol. 26, 1987; A.R. FRITZBERG, pp. 13-18&NUM;**

(73) Proprietor: **MALLINCKRODT, INC.**
**Post Office Box 5840**
**St. Louis, MI 63134(US)**

(72) Inventor: **EISENHUT, Michael, Klinikum der Univ. Heidelberg**
**Radiologische Klinik,**
**Abt. Nuklearmedizin**
**Neuenheimer Feld 400, D-6900 Heidelberg(DE)**
Inventor: **BRANDAU, Wolfgang, Klinikum der Univ. Heidelberg**
**Radiologische Klinik,**
**Abt. Nuklearmedizin**
**Neuenheimer Feld 400, D-6900 Heidelberg(DE)**

(74) Representative: **Eyles, Christopher Thomas et al**
**W.P. THOMPSON & CO.**
**High Holborn House**
**52-54 High Holborn**
**London WC1V 6RY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to a method of preparing a diaminedithiol chelating agent, new $N_2S_2$ compounds, and kits for preparing compositions containing these compounds.

Some years ago Kung et al. (J. Nucl. Med. 25, 326-332, 1984) synthesized some derivatives of the bis-aminoethane-thiol (BAT) ring system to be used as tetradentate ligands for complexing metal ions, in particular Tc-99m for brain imaging. The same compounds are described in European patent application 200211, published in 1986. Other BAT derivatives and their complexing ability are the subject of publications of Burns et al. (European pat. application 163119, published in 1985; J. Nucl. Med. 26, 1287-1294, 1985) and of Efange et al., J. Nucl. Med. 28, 1012-1019, 1987. The last-mentioned BAT derivatives having functional side-groups are especially designed to be capable, after having been complexed with Tc-99m, to cross the blood-brain barrier and then to be retained by the brain for sufficient time to permit radioassaying thereof.

All these known BAT derivatives, also called $N_2S_2$-compounds because of their tetradentate liganding capacity wherein two N-atoms and two S-atoms per molecule are involved, are bis(tertiary thiols), i.e. the thiol or mercapto groups are attached to tertiary carbon atoms. Apparently such structures are necessary to enable the Tc-99m complexes to reach the target organ, e.g., the brain, without metabolic decomposition. In the preparation of these known BAT derivatives or $N_2S_2$-compounds the formation of the two thiol functions by a reductive cleavage of the corresponding disulfide bond is always the last reaction step. It stands to reason, that the yield in this last reaction step is of paramount importance. As a matter of fact, the disulfide compound, starting substance for this reductive cleavage, has been obtained after a generally complex and consequently expensive series of reaction steps which ultimately lead to the desired side chain functionalized compound. If the reductive cleavage produces the final dithiol compound in an insufficient yield, not only is expensive material lost but also the final dithiol compound needs an extensive and laborious purification to achieve a product with a pharmaceutically acceptable purity.

Said reductive cleavage of tertiary disulfides is investigated in more detail by Corbin and Work: J. Org. Chem. Vol. 41. No. 3. 1976, 489-491. Corbin and Work disclose the resistance of "tertiary disulfides" to reduction. These authors recommend a reduction with $LiAlH_4$ in refluxing tetrahydrofuran "as long as ..... reductant-compatible substituents are present". Such reductive conditions cause a serious limitation of the side chain designed for functionalizing the $N_2S_2$-compound, because many of such side chains, e.g. carbonyl-containing groups, or protein or proteinaceous side chains, generally are not resistant to $LiAlH_4$ and/or to refluxing tetrahydrofuran. In addition, the yield of this reductive cleavage is generally rather poor. The authors mentioned hereinbefore report yields of approximately 50%, apparently dependent on the structure of the starting disulfide occasionally exceeding 60%.

It is one object of the present invention to provide a method of preparing a diaminedithiol chelating agent by reductive cleavage of the corresponding ditertiary disulfide, i.e. a disulfide wherein the sulphur atoms are attached to tertiary carbon atoms, in which the desired product is obtained in an improved yield. It has been found, that this object can be achieved, in that a compound of the general formula

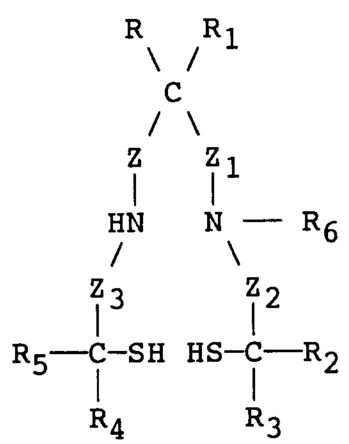

(I)

wherein

Z, $Z_1$, $Z_2$ and $Z_3$ may be the same or different and represent methylene groups, optionally substituted with one or two $C_1$-$C_4$ alkyl group(s), or carbonyl groups;

R is a hydrogen atom or a $C_1$-$C_{16}$ hydrocarbyl group, optionally substituted with a pharmaceutically acceptable target specific group capable of enabling a metal complex formed from the chelating agent to accumulate in the target organ and derived by amine H elimination from a compound selected from a protein or proteinaceous substance, a receptor binding agent and an amino compound, or with a group permitting derivatization to a target specific group, said group permitting derivatization being selected from a halogen atom, said halogen atom being a chlorine atom, a bromine atom or an iodine atom, an aldehyde group, a carboxy group and an activated ester group;

$R_1$ is a hydrogen atom or a $C_1$-$C_{16}$ hydrocarbyl group, optionally substituted with a said pharmaceutically acceptable target specific group or with a said group permitting derivatization to said target specific group;

$R_6$ is a hydrogen atom or a $C_1$-$C_4$ alkyl group; and

$R_2$, $R_3$, $R_4$ and $R_5$ may be the same or different and represent $C_1$-$C_4$ alkyl groups;

is prepared by reacting a cyclic diamine disulfide of the general formula

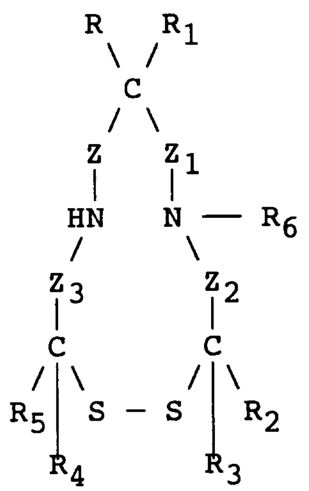

(II)

wherein Z, $Z_1$, $Z_2$, $Z_3$, R, $R_1$, $R_2$, $R_3$, $R_4$, $R_5$ and $R_6$ have the above meanings, with a thiol compound as a reducing agent, said thiol compound being selected from dithiothreitol, dithioerythritol, thioglycol, cysteine, N-acetylcysteine, glutathione, $\alpha$-mercapto-propionyl-glycine, 4-mercaptopyridine, 2,3-dimercapto-1-propanol, 1,4-dimercaptobutane and 1,3-dimercaptopropane.

Inukai et al (Bull. Chem. Soc. Japan, Vol. 40, 1967, pp. 2913-2918) have described the preparation of glutathione by the reductive cleavage of the corresponding S-ethylmercapto compound with thiophenol or thioglycollic acid: after 15 hours at 45°C the yield is nearly quantitative. Upon using thiophenol for the reductive cleavage of ditertiary disulfides according to the invention, however, a mixture of products is obtained from which the desired diaminodithiol compound cannot be isolated; this is illustrated in the Examples.

The term "pharmaceutically acceptable target specific group" will be explained hereinafter. Suitable groups permitting derivatization to target specific groups include in addition to halogen atoms (i.e. chlorine, bromine or iodine atoms), aldehyde groups, carboxy groups, and activated ester groups such as N-hydroxysuccinimidyl groups, haloacyl groups, isocyanato groups, isothiocyanato groups, trichloro-s-triazinyl groups, chlorosulphonyl groups, alkoxycarbimidoyl groups and alkylcarbonyloxy-carbonyl groups. Halogen atoms (i.e. chlorine, bromine or iodine atoms) have proved to be particularly suitable for permitting derivatization; of these halogen atoms bromine is to be preferred. In addition carboxy groups have proved to be favourable groups permitting derivatization, in particular to groups derived from a protein or proteinaceous substance, by a condensation reaction with the amino functions thereof. In a preferred configuration said carboxy group is attached to the hydrocarbyl group via a cyclic structural moiety. Of the above-mentioned reducing agents dithiothreitol and dithioerythritol are to be preferred, because by using these agents the desired reduction can be carried out in a very high yield when using moderate reaction conditions. Yields of over 95% can easily be obtained at ambient reaction temperatures. Although the above-mentioned dithiothreitol, its isomeric dithioerythritol and the other thiols are well-known reducing agents, e.g. for protein disulfide reductive cleavage, it is quite a surprise that these thiols are capable of reducing reduction-resistant tertiary disulfides under so moderate reaction conditions.

The present invention further relates to an $N_2S_2$-compoundto be used for preparing a complex for diagnostic or therapeutic application.

Frequently used diagnostic compositions comprise radionuclide-labelled compounds. Such compounds are used for diagnostic examination, e.g into deviations in shape and function of internal organs and into the presence and location of pathological processes in the body. For this purpose, a composition in which the radioactive compound is present is administered to the patient, for example, in the form of an injectable liquid. By means of a suitable detector, e.g. a gamma camera, images can be obtained by recording the emitted radiation of, for example, the organ or the pathological process in which the radioactive compound has been incorporated or is involved. Radiotherapeutic compositions are injectable compositions comprising a radioactive compound for radiotherapeutic application. It is in the purpose of this radioactive compound to emit a suitable radiation, preferably beta-rays, after incorporation in the target organ or tissue, generally a malignant tumour. By this irradiation the tumour can be eliminated or its growth can be prevented.

The above radioactive compounds or agents have one characteristic in common in that they are administered in very low dosages to achieve the desired purpose, viz. to enable a diagnostic examination or to irradiate the target organ or tissue without causing adverse side-effects. Administration of radiodiagnostic agents in larger quantities than the minimal dosages needed for imaging enhances the risk of accumulation of these agents in other places of the body than in the target organ or tissue, as a consequence of which the concentration of the agent in the environment of said target organ or tissue is increased. These background disturbances may have a serious impact on the examination of the target organ or tissue due to a decreased contrast between target organ and environmental tissue. In addition, when using radionuclide-labelled compounds, accumulation of radioactivity in other organs and tissues than the organ or tissue to be examined constitutes an extra radiation burden for these other organs and tissues which may adversely influence their health and proper functioning. This last-mentioned problem applies even more strongly to radiotherapeucic compounds, which compounds are only intended to be vehicles for carrying the radiation dose to the target organ or tissue, in particular a malignant tumour.

It will be evident from the above explanation that in particular the "target organ specificity" is of utmost importance for the above compounds or agents to be used in diagnostic or radiotherapeutic compositions. By the term "target organ specificity" is to be understood the selective presence of the compound in question in the target organ or tissue (i.e. compared to other organs or tissues) during a predetermined well-defined period of time. This latter requirement means that the compound is carried along to and accumulated in the target organ or tissue sufficiently fast and that its residence time in said organ or tissue is sufficiently long to allow a diagnostic examination or, for a radiotherapeutic compound, to make an optimum use of its radiation potential.

To impart to an $N_2S_2$-compound -to be used for preparing a complex for diagnostic or therapeutic application- an optimum "target organ specificity", viz. an optimum affinity for incorporation and accumulation in the target organ, e.g. in the brain, in an inflamed organ or tissue or in a tumour, said compound should generally be provided with a suitable functional group or side chain, which group or side-chain should be pharmaceutically acceptable. It is a disadvantage of the BAT derivatives published by the authors mentioned above, that such functionalizing comprises a plurality of subsequent reaction steps. In other words, the desired functionality cannot be introduced into the compound starting from one and the same versatile intermediate. It is another disadvantage of these known compounds, that the introduction of a functional group suitable for the desired target organ specificity involves a very laborious synthesis. Moreover, the functionalizing achieved by Burns et al involves the substitution of an H atom of one of the secondary amine functions, making the compound so obtained less suitable for complexing metal ions like Tc-99m. Anyhow, a functionality perfectly attuned to the desired target organ belongs to the basic aims in designing $N_2S_2$-compounds. In case of an insufficient "target organ specificity" or a defective pharmacokinetic behaviour a compound may remain too long in the body, beyond time after its action has been finished or the examination has been performed, and thus contributes an unnecessary burden for the patient, or, on the contrary, may have left the body too fast to do its job in a proper way. An insufficient "target organ specificity" is especially considered as a disadvantage if the compounds are intended to be used for function examination.

It is another, and indeed a very important object of the present invention to provide an $N_2S_2$-compound which can be prepared very easily by a simple reaction starting from a versatile intermediate. It has been found, that this object can be achieved by a new $N_2S_2$-compound having the general formula

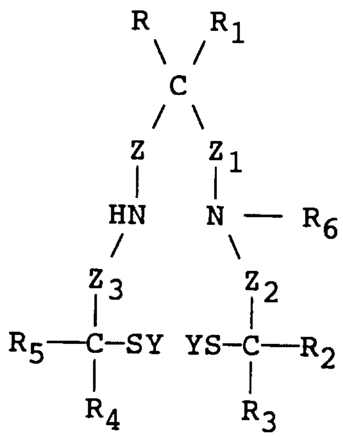

(III)

wherein

Z, $Z_1$, $Z_2$, $Z_3$, R, $R_1$, $R_2$, $R_3$, $R_4$, $R_5$ and $R_6$ have the meanings given above; and

Y is a hydrogen atom or a protective group;

or a salt of this compound with a mineral or organic acid. In case a compound is desired having one or two target specific groups, this new compound can easily be prepared from the readily available corresponding compound permitting derivatization, e.g. a halo-compound or dihalo-compound, by a simple chemical reaction, as will be explained hereinafter, of course followed by the reductive cleavage of the disulfide bond to the desired dithiol as described hereinbefore.

Examples of suitable protective groups Y for the thiol groups are: acetyl, trifluoroacetyl, hydroxyacetyl, carboxyacetyl, acetamidomethyl, benzoyl, benzyl, and benzoylaminomethyl.

A salt of the above compound encompasses salts with various acids such as hydrochloric acid, sulphuric acid, phosphoric acid, perchloric acid and organic acids like citric acid, and tartaric acid.

If a compound is desired having at least one pharmaceutically acceptable target specific group, such group is designed to impart to the compound -and consequently to the metal complex to be prepared therefrom- an optimum "target organ specificity" and pharmacokinetic behaviour. This means that such a target specific group should be attuned to the task to be performed by the compound -and its complex- in the body. So the compound functionalized in a proper way should transport the complexed metal fast and selectively via the blood stream to the target organ, e.g. the brain, easily cross the blood-target organ barrier, if present, e.g. the blood-brain barrier, and thereupon should be accumulated in said organ for sufficient time to perform its therapeutic function or to enable the user to carry out the desired examination. It stands to reason that the chemical character of such a pharmaceutically acceptable target specific group may vary widely. Examples of such groups will be disclosed hereinafter.

Dependent on the substitution pattern of the $N_2S_2$-compound, these new compounds of the present invention may occur in stereoisomers. Of course, mixtures of these stereoisomers are possible in all ratios. Optionally, these stereoisomers can be separated from each other by techniques known for this purpose, such as recrystallisation and/or chromatographic methods. The biological behaviour of the compound may be influenced by the steric configuration.

In the above formula III preferably the symbols Z, $Z_1$, $Z_2$ and $Z_3$ are methylene groups, and $R_1$ and $R_6$ are hydrogen atoms. The preferred new $N_2S_2$-compound according to the invention has the general formula

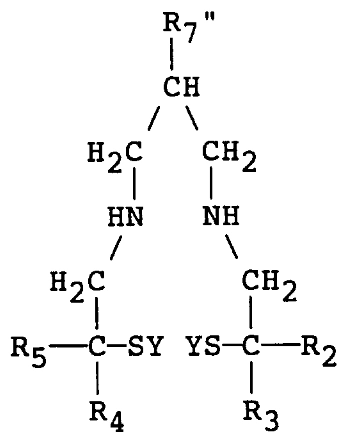

(IV)

wherein

$R_2$, $R_3$, $R_4$ and $R_5$ have the meanings given above, Y has the meaning given above, and

$R_7$" is a $C_1$-$C_{16}$ hydrocarbyl group, substituted with a halogen atom, said halogen atom being selected from a chlorine atom, a bromine atom, and an iodine atom, with a carboxy group, with an activated ester group, or with a pharmaceutically acceptable target specific group capable of enabling a metal complex formed from the $N_2S_2$-compound to accumulate in the target organ and derived from a compound selected from a protein or proteinaceous substance, a receptor binding agent, and an amino compound;

or a salt of this compound with a mineral or organic acid.

In such a compound $R_7$" may be a $C_1$-$C_{16}$ hydrocarbyl group substituted with a pharmaceutically acceptable target specific group derived from a receptor binding agent which is a compound of the formula

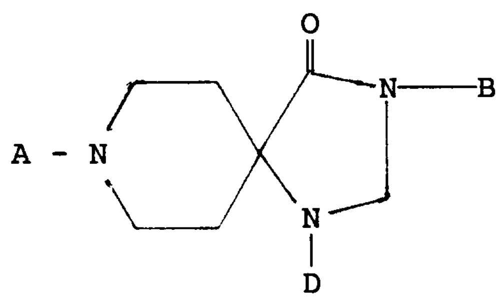

wherein A is $(CH_2)_3.CO.C_6H_4.F$-4, H, $(CH_2)_3.N(C_6H_4.F\text{-}H)_2$, or $(CH_2)_3.NH.C_6H_4.F$-4; B is H or $CH_3$; and D is $C_6H_5$ or H. If $R_7$" is a hydrocarbyl group substituted with a halogen atom, this compound can be used as an intermediate for the synthesis of a compound having a carboxy comprising group or of a compound having a pharmaceutically acceptable target specific group, by substitution of said halogen atom. A bromine atom is preferred because bromoalkyl compounds can easily be prepared from readily available raw materials and can conveniently be substituted. The hydrocarbyl group is to be considered as a spacer, spacing the functional group from the chelating compound carrying the metal ion or atom. Said spacing hydrocarbyl group may be an alkyl group, an aryl group or an aralkyl group, and may comprise a varying number of carbon atoms. Compounds having fragments derived from proteins or proteinaceous substances, for example blood corpuscles, immunoglobulins, glycopeptides, monoclonal antibodies like antimyosine and monoclonals against tumour-antigens, and other suitable proteins like plasmine and plasmine derivatives, e.g. miniplasmine and tissue-plasminogen activator, open new perspectives for preparing radiodiagnostics, in particular for the imaging of tumours, thrombi, inflammations and cardiac diseases. Certain proteins, e.g. the glycopeptide bleomycine, may be useful for the preparation of radiotherapeutic agents. The presence of amine functions in proteins or proteinaceous substances offers the opportunity to incorporate functional groups derived therefrom by a condensation reaction wth carboxy compounds, in particular with compounds of the general formula IV wherein R7" is a hydrocarbyl group substituted with a carboxy comprising group. In said carboxy containing group the carboxy group is preferably attached to a cyclic structural moiety, like a phenyl group or a saturated or unsaturated heterocyclic moiety.

A suitable example of a receptor binding agent is spiperone. Spiperone is a triamine having the formula VIII below. Spiperone, modified spiperone and spiperone derivatives, like the compounds IX, X, XI, XII and XIII, presented below, are to be considered as promising tools in imparting to the compounds of the invention favorable properties for the preparation of potential brain imaging agents.

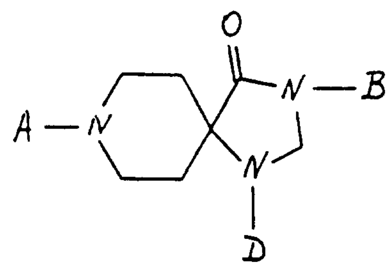

| compound | A | B | D |
|---|---|---|---|
| VIII: spiperone | $(CN_2)_3.CO.C_6H_4.F$-4 | H | $C_6H_5$ |
| IX | H | H | $C_6H_5$ |
| X | $(CH_2)_3.CO.C_6H_4.F$-4 | H | H |
| XI | $(CH_2)_3.CO.C_6H_4.F$-4 | $CH_3$ | $C_6H_5$ |
| XII | $(CH_2)_3.N(C_6H_4.F\text{-}4)_2$ | H | $C_6H_5$ |
| XIII | $(CH_2)_3.NH.C_6H_4.F$-4 | H | $C_6H_5$ |

Another example of a receptor binding agent is guanidine. The above-mentioned receptor binding agents can also be considered as amino compounds. Special amines are suitable for brain perfusion examination. In case tertiary amines are used for derivatization, quaternary ammonium compounds may be formed, which may be used for the preparation of radiodiagnostics for the localisation of neuroectodermal tumours.

In case $R_7''$ in the above formula IV is an hydrocarbyl group substituted with a pharmaceutically acceptable target specific group, said hydrocarbyl group is preferably substituted in its terminal position. If a group derived from a primary or secondary amine is used as a target specific group, said amino group may be synthesized by a simple substitution reaction from the corresponding compound having a halogen atom, preferably a bromine atom. Dependent on the chemical structure of the amine used, compounds for the preparation of radiodiagnostics suitable for a radiological examination of a variety of different organs or pathological processes can so be obtained. For such applications in addition to the above spiperone or spiperone-derived or -modified compounds IX, X, XII and XIII the following amines are to be considered: ammonia; a N-alkylamine; a N,N-dialkylamine, the alkyl groups of which together with the nitrogen atom to which they are bound may constitute a five- or six-membered heterocyclic ring, which ring may additionally comprise an oxygen atom and/or may be substituted with alkyl, amino, alkylamino, or optionally substituted phenylamino or phenylalkylamino; a N-(N',N'-dialkylaminoalkyl)-N-alkylamine; a N-(optionally substituted)-phenylalkylamine; a N-alkyl-N-(optionally substituted)phenylalkylamines. If in the above compounds mention is made of substituted phenyl, the substituents for the phenyl group may be selected from various atoms and groups, preferably from the group consisting of halogen, alkyl, alkoxy, alkylcarbonyl, alkoxycarbonyl, hydroxy, amino, alkylamino and dialkylamino. In the above compounds the alkyl groups generally comprise 1-4 carbon atoms. Some examples of such amine-derived functional groups $R_7''$ in the above formula IV are aminobutyl, N-isopropylaminobutyl, N,N-dimethylaminobutyl, N,N-diethylaminobutyl, N-piperidylbutyl, N-morpholinylbutyl, N-p-methylpiperidylbutyl, N-p-propylpiperidylbutyl and 1-phenyl-1,3,8-triazaspiro(4,5)-decan-4-on-8-ylbutyl. The above starting compound, viz. the compound of the above formula IV wherein $R_7''$ is a halogen substituted hydrocarbyl group, is also very suitable for the synthesis of carboxy group comprising compounds. In this manner various compounds can be prepared which are capable of reacting with amine functions in proteins and proteinaceous material. Examples of such compounds having a carboxy group are compounds of the above formula IV wherein $R_7''$ is N-p-carboxypiperidylbutyl or p-carboxyphenoxybutyl.

The present invention further relates to a compound to be used for the reductive cleavage as described hereinbefore having the general formula

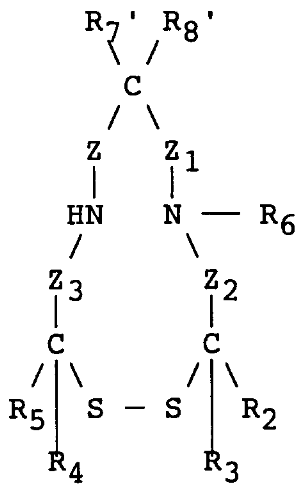

(VI)

wherein

Z, $Z_1$, $Z_2$, $Z_3$, $R_2$, $R_3$, $R_4$, $R_5$ and $R_6$ have the meanings given above,

$R_7'$ represents a $C_1$-$C_{16}$ hydrocarbyl group, substituted with a pharmaceutically acceptable target specific group capable of enabling a metal complex formed from the chelating agent formed by reduction of the disulfide bond in the compound of formula (VI) to accumulate in the target organ and derived from a compound selected from a protein or proteinaceous substance, a receptor binding agent and an amino compound; and

$R_8'$ is a hydrogen atom or a $C_1$-$C_{16}$ hydrocarbyl group, optionally substituted with a said pharmaceutically acceptable target specific group.

When $R_7'$ represents a $C_1$-$C_{16}$ hydrocarbyl group, substituted with a pharmaceutically acceptable target specific group, derived from an amino compound, $R_7'$ is preferably substituted with a group derived (by amine-H elimination) from a primary or secondary amine selected from ammonia; a N-alkylamine; a N,N-dialkylamine, the alkyl groups of which together with the nitrogen atom to which they are bound may constitute a five- or six-membered heterocyclic ring, which ring may additionally comprise an oxygen atom and/or may be substituted with alkyl, amino, alkylamino or optionally substituted phenylamino or phenylalkyl-amino; a N-(N',N'-dialkylaminoalkyl)-N-alkylamine; a N-(optionally substituted) phenylalkylamine; a N-alkyl-N-(optionally substituted) phenylalkylamine; and a compound of formula

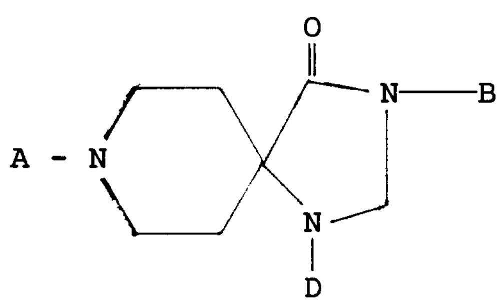

wherein A, B and D are as defined above; and wherein all of the above-mentioned alkyl groups comprise 1-4 carbon atoms. This method can be performed by reacting a precursor of the general formula

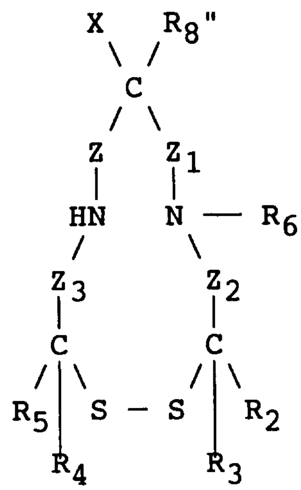

(VII)

wherein

Z, $Z_1$, $Z_2$, $Z_3$, $R_2$, $R_3$, $R_4$, $R_5$ and $R_6$ have the meanings given above,

X is a $C_1$-$C_{16}$ hydrocarbyl group substituted with a group permitting derivatization to a said target specific group, said group permitting derivatization being selected from a halogen atom, said halogen atom being selected from a chlorine atom, a bromine atom, and an iodine atom, an aldehyde group, a carboxy group and an activated ester group; and

$R_8''$ is a hydrogen atom or a $C_1$-$C_{16}$ hydrocarbyl group, optionally substituted with a said group permitting derivatization to a said target specific group;

or a salt of this compound with a mineral or organic acid, with a compound comprising said target specific group. Preferably the compound comprising said target specific group is an amine, or a protein or proteinaceous substance.

The above compound VII is a very versatile intermediate for the preparation of chelating agents or ligands functionalized with a suitable target specific side chain. After derivatization, e.g. by the above reaction with a suitable primary or secondary amine or with a protein, the compound obtained, having the general formula VI, can easily be subjected to a reductive disulfide cleavage as described hereinbefore.

The present invention also relates to a method of preparing a complex of a metal, which metal is selected from radionuclides, metals of the 7th and 8th subgroup, and lanthanides, with a chelating agent, said complex being destined for diagnostic or therapeutic application, by bringing a salt or chelate of said metal into a chelating reaction with the new $N_2S_2$-compound as defined hereinbefore.

Suitable metal-radionuclides to be used for the above preparation method according to the invention are, for example, Tc-99m, Re-186, Re-188, Cu-67, Pb-203, Pb-212, Ga-67, Ga-68, Bi-212, As-72, As-77, In-111, In-113m, Ru-97, Y-90, Ag-111 and Pd-109. From these radionuclides Tc-99m, Pb-203, Ga-67, Ga-68, As-72, In-111, In-113m and Ru-97 can be used for diagnostic purposes, the other ones are particularly useful in therapeutically effective compositions. The above method of preparing the desired complex can generally be carried out in a simple manner, preferably in a substantially aqueous medium at a substantially neutral pH (6.5-8). For the complex formation the desired metal is offered to the chelating agent in the form of a salt or in the form of a chelate wherein the metal is bound to relatively weak chelators, such as a pyrophosphate, a phosphonate or polyphosphonate, a polyphosphate, an oxinate, a carboxylate, a hydroxycarboxylate, an aminocarboxylate, an enolate or a mixture thereof. In using a metal chelate as starting material for the complex formation, the desired complex is formed via the principle of ligand exchange, the $N_2S_2$-compound providing a tetradentate ligandation of the metal.

The invention also relates to a radiotherapeutic composition. A suitable radio therapeutic composition according to the invention comprises as the active ingredient a complex carrying a beta-emitter or another radionuclide suitable for radiotherapy. Suitable radionuclides for radiotherapy are e.g. the radionuclides listed in "Radionuclides for Therapy", ed. by P.A. Schubiger and P.H. Hasler, June 13-14, 1986. Said complex is, according to the invention, prepared according to the chelating reaction as described above.

The invention further relates to a radiodiagnostic composition, comprising in addition to a pharmaceutically acceptable liquid carrier medium a complex of a radionuclide as defined hereinbefore. Said complex is, according to the invention, prepared as described above, viz. by bringing a salt or chelate of the metal into a chelating reaction with the new $N_2S_2$-compound defined hereinbefore. If desired the solution so obtained can be brought into a form more suitable for intravenous or subcutaneous application, e.g. by a

purification or by adding a pharmaceutically acceptable liquid carrier material. For intravenous or subcutaneous application the solution should of course be in a sterile condition.

For performing a radiodiagnostic examination the composition, as described above, if desired after dilution with a pharmaceutically acceptable liquid, preferably a physiological saline solution, can be administered to a warm-blooded living being in a quantity sufficient for detection by means of external imaging, preferably from 0.1 to 30 millicurie per 70 kg of body weight. Thereupon the being is subjected to external imaging to detect accumulated radioactivity. This enables the subsequent location of the accumulated radioactivity in the body of the being.

A composition destined for a radiotherapeutic treatment comprises as the active ingredient a complex of a metal nuclide suitable for this purpose; this has been explained above. Upon use this composition, if desired after dilution with a pharmaceutically acceptable liquid, is administered to a warm-blooded living being in a quantity effective for combating or controlling tumours, in particular malignant tumours.

In connection with the often poor shelf life of the radiolabelled compound and/or the short half-life time of the metal radionuclide used it is frequently impossible to put the ready-for-use composition at the disposal of the user. In such cases the user will carry out the labeling reaction with the radionuclide in the clinical hospital or laboratory. For this purpose the various reaction ingredients are then offered to the user in the form of a so-called "kit" formulation. It will be obvious that the manipulations necessary to perform the desired reaction should be as simple as possible to enable the user to prepare from the kit the radioactive labelled composition by using the facilities that are at his disposition. Because the radiopharmaceutical composition according to the present invention can be prepared in a very simple and easy manner, this preparation process can be carried out very well by the user. Therefore the invention also relates to a kit for preparing a radiopharmaceutical composition, as described above, comprising (i) in an optionally dry condition a preparation of the new $N_2S_2$ compound as described hereinbefore, to which, if desired, an inert pharmaceutically acceptable carrier and/or one or more formulating agents and/or one or more auxiliary substances is/are added, (ii) a solution of a salt or chelate of a metal radionuclide, and (iii) if desired, instructions for use with a prescription for bringing (ii) in a chelating reaction with (i) as described hereinbefore. As mentioned before, for said chelating or complexing reaction the desired neutral radionuclide may be offered to the chelating agent in the form of a chelate, bound to a relatively weak chelator, such as a pyrophosphate, a polyphosphate, a phosphonate or polyphosphonate, an oxinate, a carboxylate, a hydroxycarboxylate, an aminocarboxylate, an enolate or a mixture thereof, said reaction being carried out under moderate conditions. Examples of suitable chelators for the radionuclide are 8-hydroxyquinoline or derivatives thereof; dicarboxylic aids, polycarboxylic acids or hydroxycarboxylic acids, such as oxalic acid, malonic acid, succinic acid, maleic acid, phthalic acid, malic acid, lactic acid, tartaric acid, citric acid, ascorbic acid, salicylic acid or derivatives of these acids; pyrophosphates; phosphonates or polyphosphonates such as methylene diphosphonate, hydroxyethylene diphosphonate or hydroxymethylene diphosphonate; or enolates, for example tropolone or enolates with a beta-diketone, such as furoylacetone, thenoylacetone, benzoylacetone, dibenzoylmethane, or derivatives of these diketones. In particular are to be considered 8-hydroxyquinoline, citric acid, tartaric acid, ascorbic acid, glucoheptonic acid or a derivative thereof as chelators, because it has appeared that a chelate of a radionuclide, for example indium-111 or lead-203, can easily be brought in a complexing reaction with the chelating agent as defined hereinbefore when using suitable conditions, preferably a buffered aqueous solution and a substantially physiological pH. Then the desired radionuclide complex can be formed by ligand exchange in a high yield and purity, the $N_2S_2$-compound of the invention serving as a tetradentate ligand encompassing the metal radionuclide completely. A buffered aqueous indium-111-tropolonate solution suitable for this purpose is described in European patent application no. 131327. The kit to be supplied to the user may also comprise the ingredient defined under (i) above together with instructions for use, if desired, whereas the solution of the metal radionuclide, defined, under (ii) above, which solution has a limited shelf life, may be put to the disposal of the user separately.

In a different, equally extremely favourable embodiment the kit according to the invention comprises (i) in an optionally dry condition a preparation of the new $N_2S_2$ compound as described hereinbefore, to which, if desired, an inert pharmaceutically acceptable carrier and/or formulating agents and/or auxiliary substances is/are added, (ii) a reducing agent and, if desired, a chelator, and (iii) if desired, instructions for use with a prescription for bringing technetium-99m in the form of a pertechnetate solution, or rhenium-186 or rhenium-188 in the form of a perrhenate solution in a chelating reaction with ingredients (i) and (ii) as described hereinbefore. The composition should comprise a reducing agent to reduce the pertechnetate, for example a dithionite or stannous ions. Such a kit is intended for preparing a pharmaceutical composition labelled with Tc-99m. The pertechnetate can be obtained by the user very simply from a molybdenumtechnetium generator. A similar kit can be used for preparing a pharmaceutical composition labelled with Re-186

or Re-188. The available perrhenate solution should also be reduced with a suitable reducing agent like a dithionite or stannous ions. If desired, the ingredients defined under (i) and (ii) above may be combined, provided they are compatible with each other.

The ingredient of both above kits mentioned under (i) may be supplied as a solution, for example in the form of an physiological saline solution, or in some buffer solution, but is preferably present in a dry condition, for example in a lyophilized condition. Upon use as a component for an injection liquid, this ingredient should be in a sterile condition. In case the ingredient is in a dry condition, the user may use a sterile physiological saline solution as a solvent therefor. If desired, the above-mentioned ingredient may be stabilized in a usual way with suitable stabilizers like ascorbic acid, gentisic acid or salts of these acids, or may be provided with other auxiliaries like fillers, e.g. glucose, lactose, mannitol, etc.

The invention will now be described in more detail with reference to the ensuing specific examples.

EXAMPLE I

Preparation of 7-(4'-bromobutyl)-3,3,11,11-tetramethyl-1,2-dithia-5,9-diazacycloundecane hydrobromide: compound XIV below.

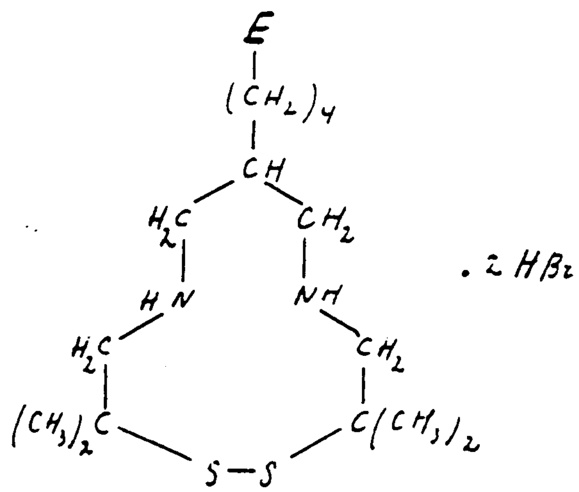

| compound | E |
|---|---|
| XIV | Br |
| XV-A | 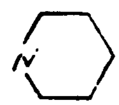 |
| XVI-A | 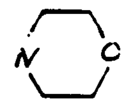 |
| XVII-A | $NH_2$ |
| XVIII-A | 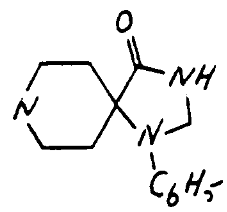 |
| XIX-A | 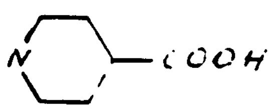 |
| XX-A | 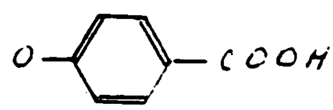 |

(a) Preparation of 2-cyano-6-phenoxyhexanoic acid nitrile.
11.87 g dry sodium hydride (0.436 mol) is suspended under argon in 100 ml dimethyl formamide, and 32.67 g malonic acid dinitrile (0.495 mol), dissolved in 100 ml DMF, are added during 3 hours with cooling to 0 ° C. Subsequently, 113.5 g 1-phenoxy-4-bromobutane (0.495 mol) dissolved in 100 ml DMF, are added within 6 hours. After stirring for an additional 6 hours at room temperature, the reaction is quenched by the addition of 1 l ice and 20 ml concentrated hydrochloric acid. Extraction with chloroform, drying of the combined organic phases with sodium sulphate and evaporation of the solvents give a dark yellow oil. Addition of 200 ml methanol results in the crystallization of 1,9-bis(phenoxy)-5,5-dicyanononane (29.7 g. 0.048 mol, 19%). Filtration and vacuum distillation of the filtrate (bp: 190 ° C, 0.5 mbar) give a colourless material which crystallizes from methanol (55.4 g, 0.258 mol; 59.3% based on NaH) as pure 2-cyano-6-phenoxyhexanoic acid nitrile; mp 53-55 ° C.
b) Preparation of 2-aminomethyl-6-phenoxy-hexylamine. 12.85 g 2-Cyano-6-phenoxyhexanoic acid nitrile (59,9 mmol) are dissolved in 1.8 l methanol. After the addition of 15 g Pd/C catalyst and 72 ml ethanolic HCl (5 N) the mixture is hydrogenated at room temperature (p = 0.1 bar) for 4 days. Filtration from the catalyst, evaporation of the filtrate and recrystallization of the residue from methanol give 14 g of the dihydrochloride as colourless needles: m.p. 202-204 ° C (decomp.). Dissolution of the dihydrochloride in a stoichiometric amount of ethanolic KOH, filtration from precipitated KCl and vacuum distillation of the filtrate results in 10.1 g 2-aminomethyl-6-phenoxy-hexylamine (45 mmol; 75%) as a colourless oil; bp: 160 ° C, 5 mbar. The oil solidifies within several months; mp: 112-117 ° C.
(c) preparation of 7-(4'-phenoxybutyl)-3,3,11,11. tetramethyl-1,2-dithia-5,9-diazacycloundecane hydrobromide 8.16 g 2-aminomethyl-6-phenoxy-hexylamine (36.7 mmol) and 7.56 g 2,2'-dithio-bis(2-methylbutanal) (36.7 mmol) are refluxed in 500 ml ethanol. The solvent is evaporated under reduced pressure and the residue redissolved in 200 ml ethanol. 5.5. g $NaBH_4$ (0.146 mmol) are added subsequently in several portions. The reduction is completed by heating the mixture for an additional

hour. The solvent is again evaporated and the residue treated with $H_2O$. $CHCl_3$-extraction affords 9.94 g crude oil (68%) which precipitates from ethanol/48% HBr as the dihydrobromide salt of the title compound (10.59 g; 51.7%), mp: >200 ° C (decomp.)

(d) Preparation of 7-(4'-bromobutyl)-3,3,11,11-tetramethyl-1,2-dithia-5,9-diazacycloundecane hydrobromide (XIV).

1.18 g of the compound prepared under (c) (2.11 mmol) is heated for 5 days at a temperature of 90 ° C in an Ar purged AcOH/48% HBr-mixture. After the evaporation of the AcOH/48% HBr-mixture the title compound is precipitated from MeOH/$CH_3CN$ (0,78 g; 68%), mp: >200 ° C (decomp.).

## EXAMPLE II

(a) Preparation of 7-(4'-N-piperidylbutyl)-3,3,11,11-tetramethyl-1,2-dithia-5,9-diazacycloundecane: compound XV-A as presented in Example I.

To 0.1 g (0.183 mmol) of the 4-bromocompound (XIV) obtained as described in Example I, dissolved in 20 ml ethanol, is added 0.1 g (1.18 mmol) of piperidine; the mixture is then stirred at ambient temperature for 24 hours. The desired alkylated amine is purified by column chromatography. The viscous product (XV-A) is obtained in a yield of 85%. The structure of the product is affirmed by mass and NMR spectroscopy.

In a comparable way the compounds presented in the table of Example I under nos. XVI-A, XVII-A, XVIII-A and XIX-A are prepared, using morpholine, ammonia, 1-phenyl-1,3-diaza-spiro(4,5)decan-4-one-8-amine and 4-carboxypiperidine as the starting amines. The products are also identified by mass and NMR spectroscopy.

| compound | yield | mass-spectral data | nmr data |
|---|---|---|---|
| XV-A | 85% | 387 ($M^+$, 40%)<br>322 ($M^+$-$S_2H$, 29%)<br>283 ($M^+$-$SC(CH_3)_2NH_2$, 27%) | 0.9-1.8 (m, 25H, $CH_3$ $CH_2$ CH)<br>2.2-3.2 (m, 14H, $CH_2$-N)<br>2.5 (s, 2H, NH) |
| XVI-A | 83% | 389 ($M^+$, 94%)<br>324 ($M^+$-$S_2H$, 17%)<br>285 ($M^+$-$SC(CH_3)_2NH_2$, 23%) | 0.9-1.6 (m, 19H, $CH_3$ $CH_2$ CH)<br>2.1-3.2 (m, 14H, $CH_2$-N)<br>2.2 (s, 2H, NH)<br>3.7-3.8 (m, 4H, $CH_2$-O) |
| XVII-A | 78% | 319 ($M^+$, 100%) | 1.0-2.1 (m, 19H, $CH_3$ $CH_2$ CH)<br>2.2-3.2 (m, 10H, $CH_2$-N)<br>2.15 (s, 4H, NH $NH_2$) |
| XVIII-A | 85% | 533 ($M^+$, 20%)<br>468 ($M^+$-$S_2H$, 12%)<br>429 ($M^+$-$SC(CH_3)_2NH_2$, 6%) | 1.0-1.8 (m, 23H, $CH_3$ $CH_2$ CH)<br>2.2-3.2 (m, 16H, $CH_2$-N)<br>2.5 (s, 2H, NH)<br>6.7-7.3 (m, 5H, $C_6H_5$) |
| XIX-A | 76% | | 0.9-1.8 (m, 23H, $CH_3$ $CH_2$ CH)<br>2.2-3.1 (m, 15H, $CH_2$-N CH-CO) |

(b) Preparation of 7-(4'-p-carboxyphenyloxybutyl)-3,3,11,11-tetramethyl-1,2-dithia-5,9-diazacycloundecane: compound XX-A as presented in Example I.

167.5 mg (1.01 mmol) p-Phenoxybenzoic acid ethyl ester was dissolved under $N_2$ in 2.5 ml DMF, cooled to 0 ° C and treated with 120 mg NaH (5 mmol). After cessation of hydrogen formation 500 mg 4-bromocompound (XIV) (0.915 mmol in 10 ml DMF) was added over a time period of 20 minutes and stirred for another 30 minutes at 0 ° C. After stirring overnight at ambient temperature the reaction was stopped by the addition of $H_2O$ and $CH_2Cl_2$. Upon cooling crystals of compound XX-A were isolated and characterized by NMR spectroscopy. Yield 72%, NMR 1.1-2.2 (m, 19H, $CH_3$ $CH_2$ CH),2.4-3.2 (m, 8H, $CH_2$-N), 3.9 (t, J = 5 Hz, $CH_2$-O) 7.35 (AA'BB', $C_6H_4$).

EXAMPLE III

(a) Reductive cleavage of 7-(4'-N-piperidylbuyl)-3,3,11,11-tetramethyl-1,2-dithia-5,9-diazacycloundecane.

Compound XV-A, obtained according to Example II, is dissolved in a quantity of 0.16 g (0.413 mmol) in 2 ml of methanol. This solution is treated at ambient temperature with 0.314 g (2.03 mmol) of 1,4-dimercapto-2,3-dihydroxybutane (dithiothreitol) for 24 hours. At the end of the reaction the mixture is acidified with HBr and extracted several times with diethylether. The oily residue solidifies under reduced pressure to an amorphous salt. The desired dithiol compound XV-B, presented below, is identified by NMR and FAB-mass spectroscopy. The yield is 95%.

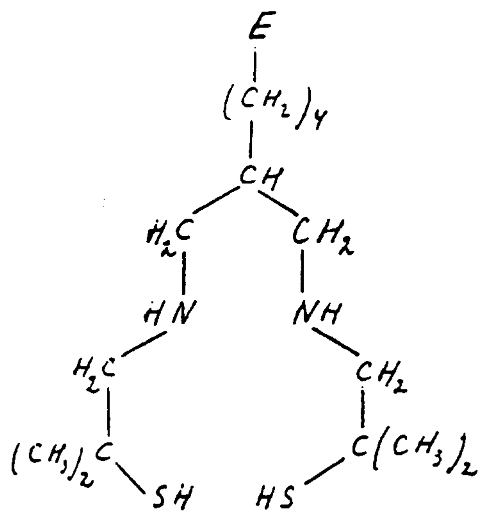

| compound | E |
|---|---|
| XV-B | 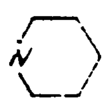 |
| XVI-B | 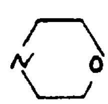 |
| XVII-B | $NH_2$ |
| XVIII-B | 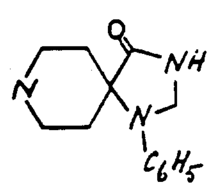 |
| XIX-B | 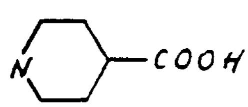 |

| compound | E |
|---|---|
| XX-B | 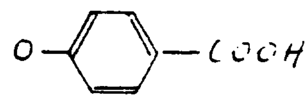 |

In a comparable way the dithiol compounds presented under nos. XVI-B, XVII-B, XVIII-B, XIX-B and XX-B are prepared.

| compound | yield | FAB mass-spectral data |
|---|---|---|
| XV-B | 95% | 390 (MH+, 48%) |
| XVI-B | 92% | 392 (MH+, 89%) |
| XVII-B | 90% | 322 (MH+, 100%) |
| XVIII-B | 92% | 536 (MH+, 76%) |
| XIX-B | 90% | 434 (MH+, 31%) |

(b) Reductive cleavage of compound XVIII-A as presented in Example I.

100 mg of compound XVIII-A (0.187 mmol), obtained according to Example II, was dissolved in 2 ml methanol. A quantity of 225 mg thiophenol was added and the solution was stirred over night at ambient temperature. Reversed phase HPLC analysis revealed a mixture of products including 30-50% of the desired ligand and 50-70% of mono- and dithiophenyl derivatives thereof. The isolation of the ligand XVIII-B from these reaction mixtures was not possible. From these results it may be concluded, that thiophenol is not suitable as a reducing agent for the desired cleavage.

EXAMPLE IV

Labelling of the diaminodithiol compound of Example III with technetium-99m.

A mixture of 5 µl tin(II) chloride (0.05 mmol/ml ethanol) and 25 µl diaminodithiolderivative hydrobromide (0.05 mmol/ml water) of the formula XV-B, prepared according to Example III, is added to 5 GBq $Na^{99m}TcO_4$ in 1 ml physiological salt solution. The pH of the solution is adjusted to 7.4 by adding 0.1 ml phosphate buffer of that pH. The solution is then made sterile by filtering it through a 0.2 µm sterile filter. The labelling yield is determined by HPLC and amounts to 80-95%.

In the same way the diaminodithiol compounds XVI-B, XVII-B, XVIII-B and XIX-B are labelled with technetium-99m in yields of 80-95%.

EXAMPLE V

A mixture of 1 GBq (0.3 ml) technetium-99m complex with diaminodithiol compound XIX-B, prepared according to Example IV, and 50 µl N-hydroxysuccinimide (0.05 mmol/ml $H_2O$) was treated with 50 µl N-(3-dimethylaminopropyl)-N'-ethylcarbodiimide (0.05 mmol/ml $H_2O$) for 1 hour at ambient temperature. Addition of 100 µl (200 mg/ml) human serum albumin (HSA) and chromatography on Sephadex G25® (2% $Et_3N$/phosphate buffer, pH 5) afforded 52% technetium-99m labelled HSA.

**Claims**

**1.** A method of preparing a diaminedithiol chelating agent, characterized in that a compound of the general formula

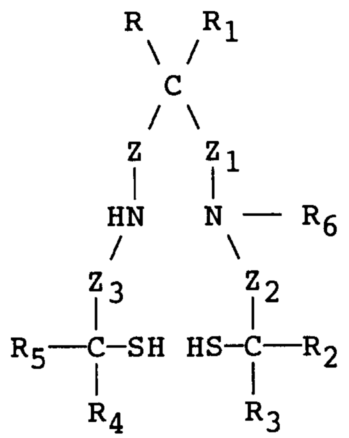

(I)

wherein

Z, $Z_1$, $Z_2$ and $Z_3$ may be the same or different and represent methylene groups, optionally substituted with one or two $C_1$-$C_4$ alkyl group(s), or carbonyl groups;

R is a hydrogen atom or a $C_1$-$C_{16}$ hydrocarbyl group, optionally substituted with a pharmaceutically acceptable target specific group capable of enabling a metal complex formed from the chelating agent to accumulate in the target organ and derived by amine H elimination from a compound selected from a protein or proteinaceous substance, a receptor binding agent and an amino compound, or with a group permitting derivatization to a target specific group, said group permitting derivatization being selected from a halogen atom, said halogen atom being a chlorine atom, a bromine atom or an iodine atom, an aldehyde group, a carboxy group and an activated ester group;

$R_1$ is a hydrogen atom or a $C_1$-$C_{16}$ hydrocarbyl group, optionally substituted with a said pharmaceutically acceptable target specific group or with a said group permitting derivatization to said target specific group;

$R_6$ is a hydrogen atom or a $C_1$-$C_4$ alkyl group; and

$R_2$, $R_3$, $R_4$ and $R_5$ may be the same or different and represent $C_1$-$C_4$ alkyl groups;

is prepared by reacting a cyclic diamine disulfide of the general formula

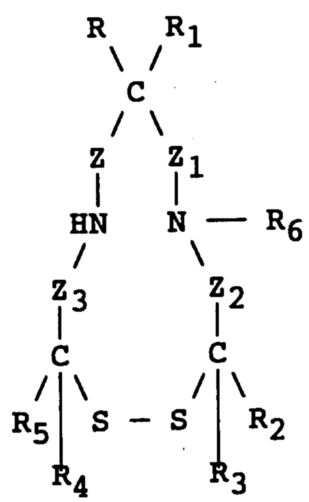

(II)

wherein Z, $Z_1$, $Z_2$, $Z_3$, R, $R_1$, $R_2$, $R_3$, $R_4$, $R_5$ and $R_6$ have the above meanings, with a thiol compound as a reducing agent, said thiol compound being selected from dithiothreitol, dithioerythritol, thioglycol, cysteine, N-acetylcysteine, glutathione, α-mercapto-propionyl-glycine, 4-mercaptopyridine, 2,3-dimercapto-1-propanol, 1,4-dimercaptobutane and 1,3-dimercaptopropane.

**2.** A method according to claim 1, characterized in that the reducing agent is dithiothreitol or dithioerythritol.

**3.** An $N_2S_2$-compound having the general formula

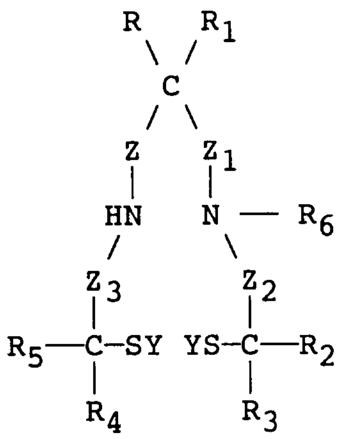

(III)

wherein
Z, $Z_1$, $Z_2$, $Z_3$, R, $R_1$, $R_2$, $R_3$, $R_4$, $R_5$ and $R_6$ have the meanings given in claim 1; and
Y is a hydrogen atom or a protective group;
or a salt of this compound with a mineral or organic acid.

**4.** A compound according to claim 3, having the general formula

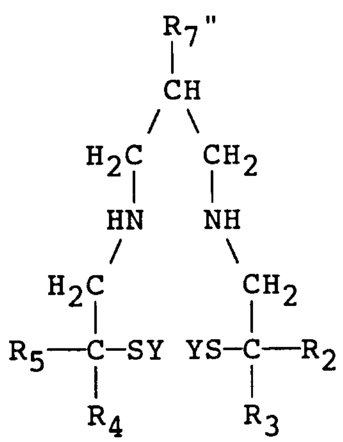

(IV)

wherein
$R_2$, $R_3$, $R_4$ and $R_5$ have the meanings given in claim 1,
Y has the meaning given in claim 3, and
$R_7''$ is a $C_1$-$C_{16}$ hydrocarbyl group, substituted with a halogen atom, said halogen atom being selected from a chlorine atom, a bromine atom, and an iodine atom, with a carboxy group, with an activated ester group, or with a pharmaceutically acceptable target specific group capable of enabling a metal complex formed from the $N_2S_2$-compound to accumulate in the target organ and derived by amine H elimination from a compound selected from a protein or proteinaceous substance, a receptor binding agent, and an amino compound;
or a salt of this compound with a mineral or organic acid.

**5.** A compound according to claim 4, in which $R_7''$ is a $C_1$-$C_{16}$ hydrocarbyl group substituted with a pharmaceutically acceptable target specific group derived from a receptor binding agent which is a compound of the formula

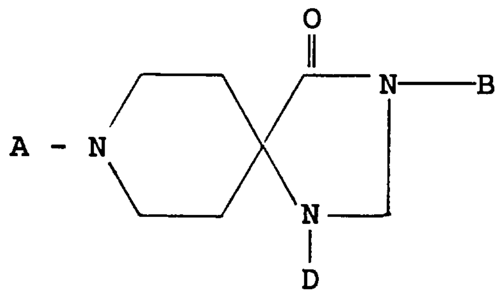

and selected from compounds of formula VIII, IX, X, XI, XII, or XIII wherein A, B and D have the meanings set out below:

| Compound | A | B | D |
|---|---|---|---|
| VIII : spiperone | $(CH_2)_3.CO.C_6H_4.F\text{-}4$ | H | $C_6H_5$ |
| IX | H | H | $C_6H_5$ |
| X | $(CH_2)_3.CO.C_6H_4.F\text{-}4$ | H | H |
| XI | $(CH_2)_3.CO.C_6H_4.F\text{-}4$ | $CH_3$ | $C_6H_5$ |
| XII | $(CH_2)_3.N(C_6H_4.F\text{-}4)_2$ | H | $C_6H_5$ |
| XIII | $(CH_2)_3.NH.C_6H_4.F\text{-}4$ | H | $C_6H_5$ |

**6.** A compound according to claim 4, having the general formula

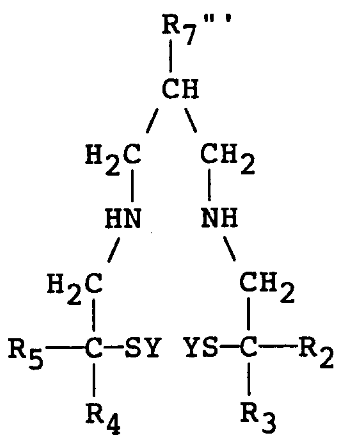

(V)

wherein

$R_2$, $R_3$, $R_4$ and $R_5$ have the meanings given in claim 1,

Y has the meaning given in claim 3, and

$R_7'''$ is a $C_1$-$C_{16}$ hydrocarbyl group, substituted with a halogen atom, said halogen atom being selected from a chlorine atom, a bromine atom, and an iodine atom, with a carboxy group, with an activated ester group, or with a group derived by amine-H elimination from a primary or secondary amine, selected from (i) ammonia; (ii) a N-alkylamine; (iii) a N,N-dialkylamine, the alkyl groups of which together with the nitrogen atom to which they are bound may constitute a five- or six-membered heterocyclic ring, which ring may additionally comprise an oxygen atom and/or may be substituted with alkyl, amino, alkylamino, or an optionally substituted phenylamino or phenylalkyl-amino; (iv) a N-(N',N'-dialkylaminoalkyl)-N-alkylamine; (v) a N-(optionally substituted) phenylalkylamine; (vi) a N-alkyl-N-(optionally substituted) phenylalkylamine; and (vii) a compound of formula

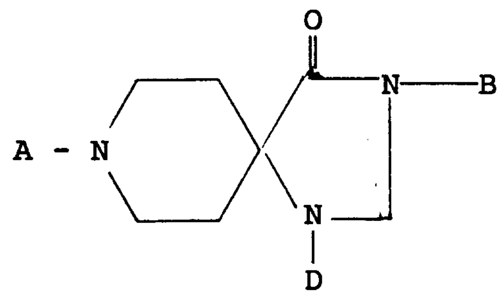

and selected from compounds of formula VIII, IX, X, XI, XII or XIII defined in claim 5; and wherein all of the above-mentioned alkyl groups comprise 1-4 carbon atoms;

or a salt of this compound with a mineral or organic acid.

**7.** A compound having the general formula

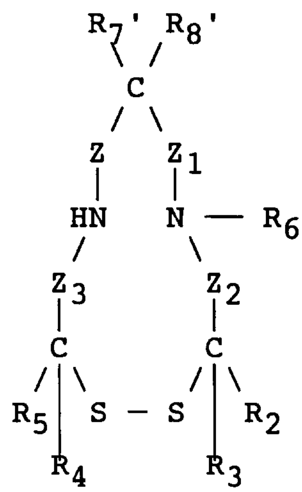

(VI)

wherein

$Z$, $Z_1$, $Z_2$, $Z_3$, $R_2$, $R_3$, $R_4$, $R_5$ and $R_6$ have the meanings given in claim 1,

$R_7'$ represents a $C_1$-$C_{16}$ hydrocarbyl group, substituted with a pharmaceutically acceptable target specific group capable of enabling a metal complex formed from the chelating agent formed by reduction of the disulfide bond in the compound of formula (VI) to accumulate in the target organ and derived from a compound selected from a protein or proteinaceous substance, a receptor binding agent and an amino compound; and

$R_8'$ is a hydrogen atom or a $C_1$-$C_{16}$ hydrocarbyl group, optionally substituted with a said pharmaceutically acceptable target specific group.

**8.** A method of preparing a compound according to claim 7, characterized in that a precursor of the general formula

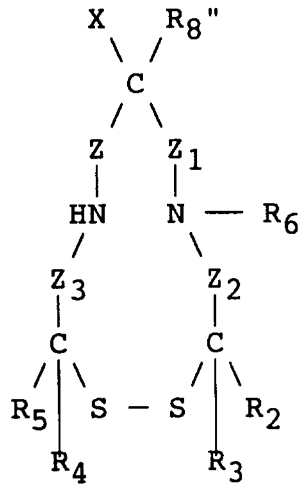

(VII)

wherein

Z, $Z_1$, $Z_2$, $Z_3$, $R_2$, $R_3$, $R_4$, $R_5$ and $R_6$ have the meanings given in claim 1,

X is a $C_1$-$C_{16}$ hydrocarbyl group substituted with a group permitting derivatization to a said target specific group, said group permitting derivatization being selected from a halogen atom, said halogen atom being selected from a chlorine atom, a bromine atom, and an iodine atom, an aldehyde group, a carboxy group and an activated ester group; and

$R_8''$ is a hydrogen atom or a $C_1$-$C_{16}$ hydrocarbyl group, optionally substituted with a said group permitting derivatization to a said target specific group;

or a salt of this compound with a mineral or organic acid, is reacted with a compound comprising said target specific group.

**9.** A method according to claim 8, characterized in that in said precursor of formula (VII) said group permitting derivatization is a halogen atom selected from a chlorine atom, a bromine atom, and an iodine atom.

**10.** A method according to claim 8 or claim 9, characterized in that said compound comprising said target specific group is an amine, or a protein or proteinaceous substance.

**11.** A complex of a metal selected from radionuclides, metals of the 7th and 8th subgroup, and lanthanides, with a chelating agent according to any one of claims 3 to 6.

**12.** A method of preparing a complex of a metal, which metal is selected from radionuclides, metals of the 7th and 8th subgroup, and lanthanides, with a chelating agent, said complex being intended for diagnostic or therapeutic application, characterized in that a salt or chelate of said metal is brought into a chelating reaction with a compound according to any one of claims 3 to 6.

**13.** A radiotherapeutic composition comprising, in addition to a pharmaceutically acceptable liquid carrier medium, a complex of a beta-emitter or another radionuclide suitable for radiotherapy, characterized in that the composition comprises a complex according to claim 11 or a complex prepared according to a method according to claim 12.

**14.** A radiodiagnostic composition comprising, in addition to a pharmaceutically acceptable liquid carrier medium, a complex of a radionuclide suitable for radioassaying, characterized in that the composition comprises a complex according to claim 11 or a complex prepared according to a method according to claim 12.

**15.** A kit for preparing a radiopharmaceutical composition, comprising (i) in an optionally dry condition a preparation of a compound according to any one of claims 3 to 6, to which, if desired, an inert pharmaceutically acceptable carrier and/or one or more formulating agents and/or one or more auxiliary substances is/are added, (ii) a solution of a salt or chelate of a metal radionuclide, and (iii), if desired, instructions for use with a prescription for bringing (ii) in a chelating reaction with (i).

**16.** A kit for preparing a radiopharmaceutical composition, comprising (i) in an optionally dry condition a preparation of a compound according to any one of claims 3 to 6, to which, if desired, an inert pharmaceutically acceptable carrier and/or one or more formulating agents and/or one or more auxiliary substances is/are added, (ii) a reducing agent and, if desired, a chelator, and (iii) if desired, instructions for use with a prescription bringing technetium-99m in the form of a pertechnetate solution, or rhenium-186 or rhenium-188 in the form of a perrhenate solution in a chelating reaction with ingredients (i) and (ii).

**17.** A method of subjecting a warm-blooded living being to a radioassay, characterized in that a composition as claimed in claim 14, if desired after dilution with a pharmaceutically acceptable liquid, is administered to the being, the quantity of administered radioactivity being sufficient for detection by means of external imaging, and that then the being is subjected to external imaging to detect accumulated radioactivity.

**18.** A method according to claim 17, wherein the quantity of administered radioactivity is from 0.1 to 30 millicurie per 70 kg of body weight.

**Patentansprüche**

**1.** Verfahren zur Herstellung eines Diamindithiol-Chelatierungsmittel, dadurch gekennzeichnet, daß eine Verbindung der allgemeinen Formel

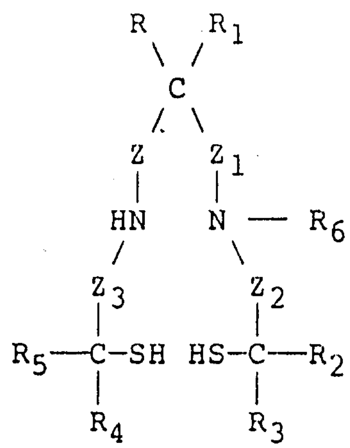

(I)

worin

Z, $Z_1$, $Z_2$ und $Z_3$ gleich oder verschieden sein können und Methylengruppen, die wahlweise mit einer oder mit zwei $C_{1-4}$-Alkylgruppe(n) substituiert sind, oder Carbonylgruppen bedeuten;

R ein Wasserstoffatom oder eine $C_1$-$C_{16}$-Kohlenwasserstoffgruppe ist, die wahlweise substituiert ist mit einer pharmazeutisch zulässigen, zielspezifischen Gruppe, die zu einem Metallkomplex aus dem Chelatierungsmittel zur Speicherung in dem Zielorgan befähigt ist und durch Aminwasserstoff-Eliminierung von einer unter einem Protein oder einer proteinartigen Substanz, einem Rezeptor-Bindungsmittel und einer Aminverbindung ausgewählten Verbindung abgeleitet ist, oder mit einer die Derivatbildung zu einer zielspezifischen Gruppe erlaubenden Gruppe, die unter einem Halogenatom, das ein Chlor-, Brom- oder Jodatom ist, einer Aldehydgruppe, einer Carboxygruppe und einer aktivierten Estergruppe ausgewählt ist;

$R_1$ ein Wasserstoffatom oder eine $C_1$-$C_{16}$-Kohlenwasserstoffgruppe ist, die wahlweise mit der genannten pharmazeutisch zulässigen zielspezifischen Gruppe oder mit der genannten, die Derivatbildung zu der genannten zielspezifischen Gruppe erlaubenden Gruppe substituiert ist;

$R_6$ ein Wasserstoffatom oder eine $C_1$-$C_4$-Alkylgruppe ist; und

$R_2$, $R_3$, $R_4$ und $R_5$ gleich oder unterschiedlich sein können und $C_1$-$C_4$-Alkylgruppen darstellen;

durch Umsetzung eines cyclischen Diamindisulfids der allgemeinen Formel

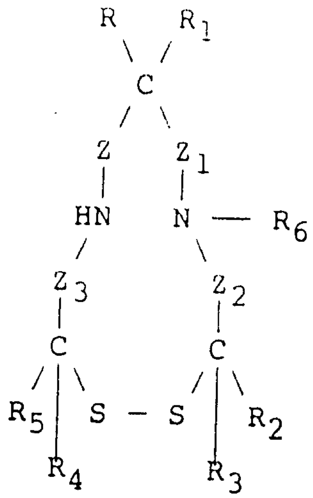

(II)

worin Z, $Z_1$, $Z_2$, $Z_3$, R, $R_1$, $R_2$, $R_3$, $R_4$, $R_5$ und $R_6$ die obigen Bedeutungen haben, mit einer Thiolverbindung als Reduktionsmittel hergestellt wird, wobei die Thiolverbindung unter Dithiothreit, Dithioerythrit, Thioglykol, Cystein, N-Acetylcystein, Glutathion, α-Merkaptopropionylglycin, 4-Merkaptopyridin, 2,3-Dimerkapto-1-propanol, 1,4-Dimerkaptobutan und 1,3-Dimerkaptopropan ausgewählt ist.

**2.** Verfahren nach Anspruch 1, dadurch gekennzeichnet, daß das Reduktionsmittel Dithiothreit oder Dithioerythrit ist.

**3.** $N_2S_2$-Verbindung mit der allgemeinen Formel

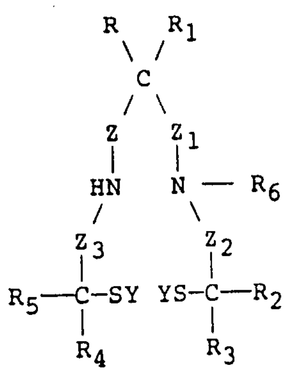

(III)

worin
Z, $Z_1$, $Z_2$, $Z_3$, R, $R_1$, $R_2$, $R_3$, $R_4$, $R_5$ und $R_6$ die in Anspruch 1 angegebenen Bedeutungen haben und
Y ein Wasserstoffatom oder eine Schutzgruppe ist,
oder ein Salz dieser Verbindung mit einer Mineralsäure oder organischen Säure.

**4.** Verbindung nach Anspruch 3 mit der allgemeinen Formel

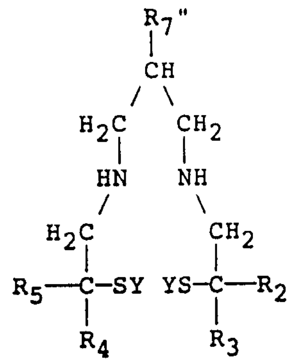

(IV)

worin

$R_2$, $R_3$, $R_4$ und $R_5$ die in Anspruch 1 angegebenen Bedeutungen haben,

Y die in Anspruch 3 angegebene Bedeutung hat, und

$R_7''$ eine $C_1$-$C_{16}$-Kohlenwasserstoffgruppe ist, die substituiert ist mit einem unter einem Chloratom, einem Bromatom oder einem Jodatom ausgewählten Halogenatom, einer Carboxygruppe, einer aktivierten Estergruppe oder mit einer pharmazeutisch zulässigen zielspezifischen Gruppe, die einen aus der $N_2S_2$-Verbindung gebildeten Metallkomplex befähigt, sich in dem Zielorgan anzusammeln, und durch Amin-H-Eliminierung von einer Verbindung abgeleitet ist, die unter einem Protein oder einer proteinartigen Substanz, einem Rezeptor-Bindungsmittel und einer Aminoverbindung ausgewählt ist;

oder sie ist ein Salz dieser Verbindung mit einer Mineralsäure oder organischen Säure.

**5.** Verbindung nach Anspruch 4, in der $R_7''$ eine $C_1$-$C_{16}$-Kohlenwasserstoffgruppe ist, die mit einer pharmazeutisch zulässigen, zielspezifischen Gruppe substituiert ist, die von einem Rezeptor-Bindungsmittel abgeleitet ist, das eine Verbindung der Formel

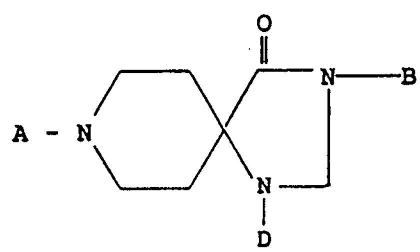

ist und aus Verbindungen der Formeln VIII, IX, X, XI, XII oder XIII ausgewählt ist, worin A, B und D die nachfolgenden Bedeutungen haben:

| Verbindung | A | B | D |
|---|---|---|---|
| VIII: Spiperon | $(CH_2)_3.CO.C_6H_4.F\text{-}4$ | H | $C_6H_5$ |
| IX | H | H | $C_6H_5$ |
| X | $(CH_2)_3.CO.C_6H_4.F\text{-}4$ | H | H |
| XI | $(CH_2)_3.CO.C_6H_4.F\text{-}4$ | $CH_3$ | $C_6H_5$ |
| XII | $(CH_2)_3.N(C_6H_4.F\text{-}4)_2$ | H | $C_6H_5$ |
| XIII | $(CH_2)_3.NH.C_6H_4.F\text{-}4$ | H | $C_6H_5$ |

**6.** Verbindung nach Anspruch 4 mit der allgemeinen Formel

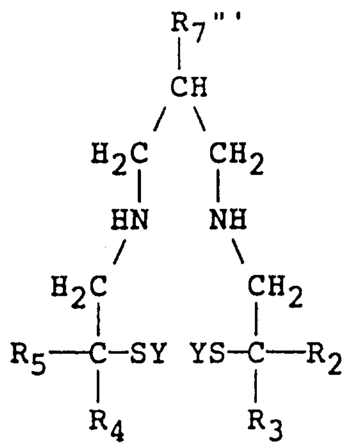

(V)

worin

$R_2$, $R_3$, $R_4$ und $R_5$ die in Anspruch 1 angegebenen Bedeutungen haben,

Y die in Anspruch 3 angegebene Bedeutung hat und

$R_7$''' eine $C_1$-$C_{16}$-Kohlenwasserstoffgruppe ist, die substituiert ist mit einem unter einem Chloratom, Bromatom und Jodatom ausgewählten Halogenatom, einer Carboxygruppe, einer aktivierten Estergruppe oder mit einer Gruppe, die die sich durch Amin-H-Eliminierung von einem primären oder sekundären Amin ableitet, das ausgewählt ist unter (i) Ammoniak, (ii) einem N-Alkylamin, (iii) einem N,N-Dialkylamin, deren Alkylgruppen zusammen mit dem Stickstoffatom, an das sie gebunden sind, einen fünf- oder sechsgliedrigen, heterocyclischen Ring bilden kann, der zusätzlich ein Sauerstoffatom enthalten kann und/oder mit Alkyl, Amino, Alkylamino oder einem wahlweise substituierten Phenylamino oder Phenylalkylamino substituiert sein kann, (iv) einem N-(N',N'-Dialkylaminoalkyl)N-alkylamin, (v) einem N-(wahlweise substituierten) Phenylalkylamin, (vi) einem N-Alkyl-N-(wahlweise substituierten) Phenylalkylamin, und (vii) einer Verbindung der Formel

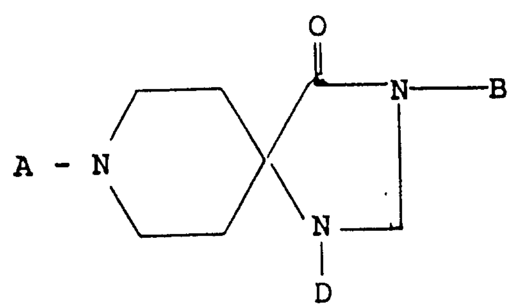

die unter den Verbindungen der in Anspruch 5 definierten Formeln ausgewählt ist, und worin alle oben erwähnten Alkylgruppen 1-4 Kohlenstoffatome enthalten,

oder ein Salz dieser Verbindung mit einer Mineralsäure oder organischen Säure.

7. Verbindung mit der allgemeinen Formel

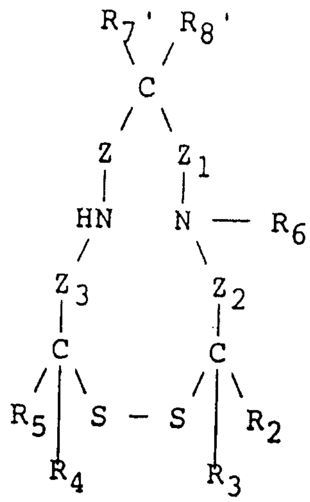

(VI)

worin

$Z$, $Z_1$, $Z_2$, $Z_3$, $R_2$, $R_3$, $R_4$, $R_5$ und $R_6$ die in Anspruch 1 angegebenen Bedeutungen haben,

$R_7'$ eine $C_1$-$C_{16}$-Kohlenwasserstoffgruppe darstellt, die mit einer pharmazeutisch zulässigen zielspezifischen Gruppe substituiert ist, welche zur Bildung eines Metallkomplexes aus dem Chelatierungsmittel befähigt ist, das zur Ansammlung in dem Zielorgan durch Reduktion der Disulfidbindung in der Verbindung der Formel (VI) gebildet wird, und die abgeleitet ist von einer unter einem Protein oder einer proteinartigen Substanz, einem Rezeptor-Bindungsmittel und einer Aminoverbindung ausgewählten Verbindung; und

$R_8'$ ein Wasserstoffatom oder eine $C_1$-$C_{16}$-Kohlenwasserstoffgruppe ist, die wahlweise mit der genannten pharmazeutisch zulässigen, zielspezifischen Gruppe substituiert ist.

8. Verfahren zur Herstellung einer Verbindung nach Anspruch 7, dadurch gekennzeichnet, daß ein Vorprodukt der allgemeinen Formel

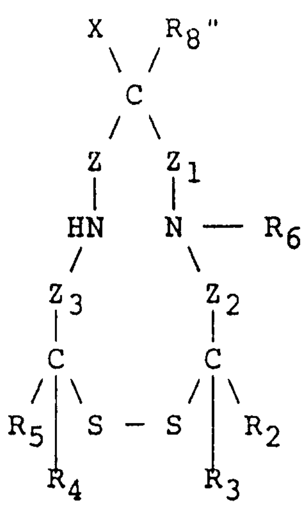

(VII)

worin

$Z$, $Z_1$, $Z_2$, $Z_3$, $R_4$, $R_5$ und $R_6$ die in Anspruch 1 angegebenen Bedeutungen haben,

X eine $C_1$-$C_{16}$-Kohlenwasserstoffgruppe ist, die mit einer die Derivatbildung zu der genannten zielspezifischen Gruppe erlaubende Gruppe substituiert ist, die unter den unter Chlor-, Brom- und

Jodatomen ausgewählten Halogenatomen, einer Aldehydgruppe, einer Carboxygruppe und einer aktivierten Estergruppe ausgewählt ist, und

$R_8$" ein Wasserstoffatom oder eine $C_1$-$C_{16}$-Kohlenwasserstoffgruppe ist, die wahlweise mit einer der genannten, die Derivatbildung zu der genannten zielspezifischen Gruppe erlaubenden Gruppe substituiert ist,

oder eines Salzes dieser Verbindung mit einer Mineralsäure oder organischen Säure mit einer die genannte zielspezifische Gruppe enthaltenden Verbindung.

**9.** Verfahren nach Anspruch 8, dadurch gekennzeichnet, daß in dem genannten Vorprodukt der Formel (VII) die die Derivatbildung erlaubende Gruppe ein Halogenatom ist, das unter einem Chloratom, Bromatom und Jodatom ausgewählt ist.

**10.** Verfahren nach Anspruch 8 oder 9, dadurch gekennzeichnet, daß die die genannte zielspezifische Gruppe enthaltende Verbindung ein Amin oder ein Protein oder eine proteinartige Substanz ist.

**11.** Komplex eines Metalls, das unter Radionukliden, Metallen der 7. und 8. Untergruppe und lanthaniden ausgewählt ist, mit einem Chelatierungsmittel nach einem der Ansprüche 3 bis 6.

**12.** Verfahren zur Herstellung eines für diagnostische oder therapeutische Anwendung vorgesehenen Komplexes eines Metalls, das unter Radionukliden, Metallen der 7. und 8. Untergruppe und Lanthaniden ausgewählt ist, mit einem Chelatierungsmittel, dadurch gekennzeichnet, daß man ein Salz oder Chelat des genannten Metalls in eine Chelatierungsumsetzung mit einer Verbindung nach einem der Ansprüche 3 bis 6 bringt.

**13.** Radiotherapeutische Zusammensetzung, die neben einem pharmazeutisch zulässigen, flüssigen Trägermedium einen Komplex eines $\beta$-Strahlers oder eines anderen für die Radiotherapie geeigneten Radionuklids enthält, dadurch gekennzeichnet, daß die Zusammensetzung einen Komplex nach Anspruch 11 oder einen nach einem Verfahren gemäß Anspruch 12 hergestellten Komplex enthält.

**14.** Radiodiagnostische Zusammensetzung, die neben einem pharmazeutisch zulässigen, flüssigen Trägermedium einen Komplex eines für die Strahlenuntersuchung geeigneten Radionuklids enthält, dadurch gekennzeichnet, daß die Zusammensetzung einen Komplex nach Anspruch 11 oder einen nach einem Verfahren nach Anspruch 12 hergestellten Komplex enthält.

**15.** Ausrüstung zur Herstellung einer radiopharmazeutischen Zusammensetzung mit (i) einem wahlweise trockenen Präparat einer Verbindung nach einem der Ansprüche 3 bis 6, dem gewünschtenfalls ein inerter, pharmazeutisch zulässiger Träger und/oder ein oder mehrere Formulierungsmittel und/oder eine oder mehrere Hilfssubstanzen zugesetzt sind, (ii) einer Lösung eines Salzes oder Chelats eines Metall-Radionuklids und (iii) gewünschtenfalls Gebrauchsanweisungen mit einer Vorschrift, wie (ii) in eine Chelatierungsumsetzung mit (i) gebracht wird.

**16.** Ausrüstung zur Herstellung einer radiopharmazeutischen Zusammensetzung mit (i) einem wahlweise trockenen Präparat einer Verbindung nach einem der Ansprüche 3 bis 6, dem gewünschtenfalls ein inerter, pharmazeutisch zulässiger Träger und/oder eine oder mehrere Formulierungsmittel und/oder eine oder mehrere Hilfssubstanzen zugesetzt sind, einem Reduktionsmittel und gewünschtenfalls einem Chelatbildner und (iii) gewünschtenfalls Gebrauchsanweisungen mit einer Vorschrift, wie Technetium-99m in Form einer Pertechnetat-Lösung oder Rhenium-186 oder Rhenium-188 in Form einer Perrhenat-Lösung in eine Chelatierungsreaktion mit den Bestandteilen (i) und (ii) gebracht wird.

**17.** Verfahren zur Durchführung einer Strahlenuntersuchung an einem Warmblüter, dadurch gekennzeichnet, daß eine Zusammensetzung nach Anspruch 14, gewünschtenfalls nach Verdünnung mit einer pharmazeutisch zulässigenFlüssigkeit, dem Lebewesen verabreicht wird, wobei die Menge der verabreichten Radioaktivität zur Feststellung durch äußere Abbildung ausreicht, und daß dann das Lebewesen einer äußeren Abbildung zur Feststellung der angesammelten Radioaktivität unterworfen wird.

**18.** Verfahren nach Anspruch 17, bei dem die Menge der verabreichten Radioaktivität 0,1 bis 30 Millicurie je 70 Kg Körpergewicht beträgt.

## Revendications

**1.** Procédé de préparation d'un agent chélateur de type diaminedithiol, caractérisé en ce qu'on prépare un composé de formule générale :

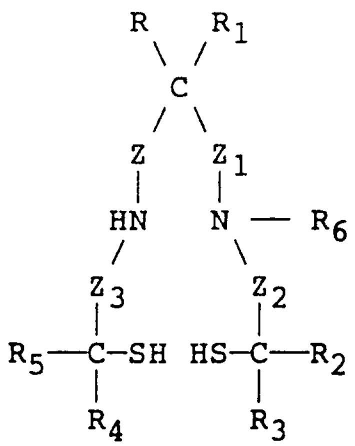

(I)

dans laquelle

Z, $Z_1$, $Z_2$ et $Z_3$ peuvent être identiques ou différents et représentent chacun des groupes méthylènes, éventuellement substituée par un ou deux groupe(s) alkyle(s) en $C_1$ à $C_4$, ou des groupes carbonyles;

R représente un atome d'hydrogène ou un groupe hydrocarbyle en $C_1$ à $C_{16}$, éventuellement substitué par un groupe pharmaceutiquement acceptable, spécifique d'une cible et capable de permettre à un complexe de métal, formé à partir de l'agent chélateur, de s'accumuler dans l'organe cible, et provenant par élimination de H d'un groupe amine, d'un composé choisi parmi une protéine ou une substance protéinique, un agent de fixation sur récepteur et une amine, ou substitué par un groupe permettant de former un dérivé donnant un groupe spécifique d'une cible, ledit groupe permettant de choisir le dérivé parmi un atome d'halogène (cet atome d'halogène étant un atome de chlore, un atome de brome ou un atome d'iode), un groupe aldéhyde, un groupe carboxy et un groupe ester activé;

$R_1$ représente un atome d'hydrogène ou un groupe hydrocarbyle en $C_1$ à $C_{16}$, éventuellement substitué par un groupe pharmaceutiquement acceptable spécifique d'une cible ou par un groupe permettant d'obtenir par formation d'un dérivé un tel groupe spécifique de cible;

$R_6$ représente un atome d'hydrogène ou un groupe alkyle en $C_1$ à $C_4$; et

$R_2$, $R_3$, $R_4$ et $R_5$ peuvent être identiques ou différents et représentent chacun un groupe alkyle en $C_1$ à $C_4$;

par la réaction d'un disulfure de diamine cyclique de formule générale

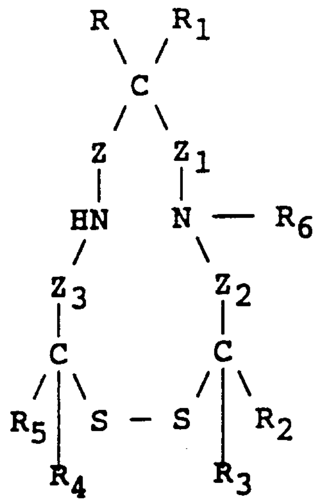

(II)

dans laquelle Z, $Z_1$, $Z_2$, $Z_3$, R, $R_1$, $R_2$, $R_3$, $R_4$, $R_5$ et $R_6$ ont les sens ci-dessus avec un thiol comme agent réducteur, ce thiol étant choisi parmi le dithiothréitol, le dithioérythritol, le thioglycol, la cystéine, la N-acétylcystéine, le glutathion, l'α-mercapto-propionyl-glycine, la 4-mercaptopyridine, le 2,3-dimercapto-1-propanol, le 1,4-dimercaptobutane et le 1,3-dimercaptopropane.

**2.** Procédé selon la revendication 1, caractérisé en ce que l'agent réducteur est le dithiothréitol ou le dithioérythritol.

**3.** Composé de type $N_2S_2$, de formule générale :

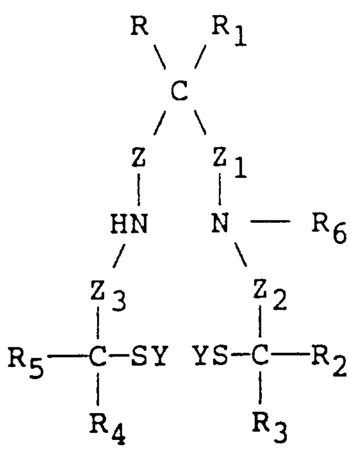

(III)

dans laquelle

Z, $Z_1$, $Z_2$, $Z_3$, R, $R_1$, $R_2$, $R_3$, $R_4$, $R_5$ et $R_6$ ont les sens indiqués à la revendication 1 ; et

Y représente un atome d'hydrogène ou un groupe protecteur;

ou un sel de ce composé avec un acide minéral ou organique.

**4.** Composé selon la revendication 3, qui a pour formule générale :

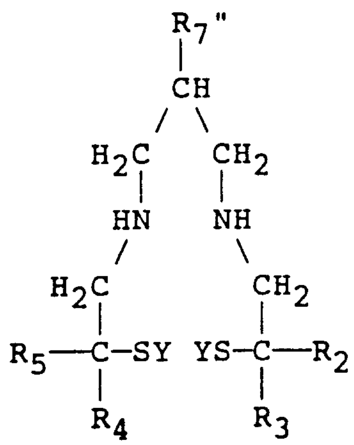

(IV)

dans laquelle

$R_2$, $R_3$, $R_4$ et $R_5$ ont les sens indiqués à la revendication 1,

Y a le sens indiqué à la revendication 3, et

$R_7$" représente un groupe hydrocarbyle en $C_1$ à $C_{16}$, substitué par un atome d'halogène (cet atome d'halogène étant choisi parmi un atome de chlore, un atome de brome et un atome d'iode), par un groupe carboxy, par un groupe ester activé ou par un groupe, pharmaceutiquement acceptable et spécifique d'une cible, permettant à un complexe de métal formé à partir du composé de type $N_2S_2$ de s'accumuler dans l'organe cible et obtenu, par élimination de H d'une fonction amine, à partir d'un composé choisi parmi une protéine et une substance protéinique, un agent de fixation sur un récepteur et une amine; ou un sel de ce composé avec un acide minéral ou organique.

**5.** Composé selon la revendication 4, dans lequel $R_7$" représente un groupe hydrocarbyle en $C_1$ à $C_{16}$ substitué par un groupe pharmaceutiquement acceptable, spécifique d'une cible, provenant d'un agent de fixation sur récepteur, qui est un composé de formule :

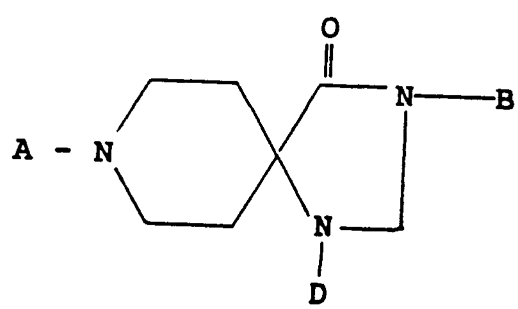

et qui est choisi parmi des composés de formule VIII, IX, X, XI, XII ou XIII, dans lesquels A, B et D ont les sens indiqués ci-après :

| Composé | A | B | D |
|---|---|---|---|
| VIII : spipérone | $(CH_2)_3.CO.C_6H_4.F\text{-}4$ | H | $C_6H_5$ |
| IX | H | H | $C_6H_5$ |
| X | $(CH_2)_3.CO.C_6H_4.F\text{-}4$ | H | H |
| XI | $(CH_2)_3.CO.C_6H_4.F\text{-}4$ | $CH_3$ | $C_6H_5$ |
| XII | $(CH_2)_3.N(C_6H_4.F\text{-}4)_2$ | H | $C_6H_5$ |
| XIII | $(CH_2)_3.NH.C_6H_4.F\text{-}4$ | H | $C_6H_5$ |

**6.** Composé selon la revendication 4, ayant la formule générale :

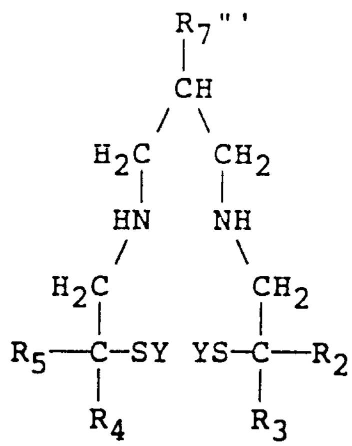

(V)

dans laquelle

$R_2$, $R_3$, $R_4$ et $R_5$ ont les sens indiqués à la revendication 1

Y a le sens indiqué à la revendication 3, et

$R_7'''$ représente un groupe hydrocarbyle en $C_1$ à $C_{16}$, substitué par un atome d'halogène (ledit atonie d'halogène étant choisi parmi un atome de chlore, un atome de brome et un atome d'iode), par un groupe carboxy, par un groupe ester activé ou par un groupe provenant, par élimination de H d'amine, d'une amine primaire ou secondaire, choisie parmi (i) l'ammoniac; (ii) une N-alkylamine ; (iii) une N,N-dialkylamine, dont les groupes alkyles peuvent former, avec l'atome d'azote auquel ils sont reliés, un noyau hétérocyclique pentagonal ou hexagonal, ledit noyau pouvant comprendre en outre un atome d'oxygène et/ou pouvant être substitué par un groupe alkyle, amino, alkylamino ou phénylamino éventuellement substitué ou phénylalkylamino ; (iv) une N-(N',N'-dialkylaminoalkyl)-N-alkylamine ; (v) une N-phényl(éventuellement substitué)-alkylamine ; (vi) une N-alkyl-N-phényl(éventuellement substitué)-alkylamine ; et (vii) un composé de formule :

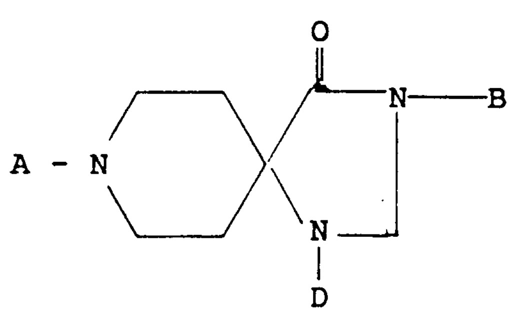

et qui est choisi parmi les composés de formules VIII, IX, X, XI, XII ou XIII définis à la revendication 5 ; et dans lequel tous les groupes alkyles précités comprennent 1 à 4 atomes de carbone;

ou un sel de ce composé avec un acide minéral ou organique.

**7.** Composé ayant la formule générale :

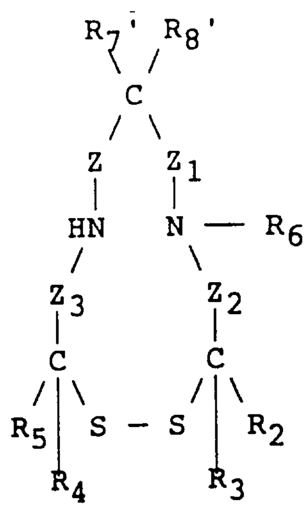

(VI)

dans laquelle

Z, $Z_1$, $Z_2$, $Z_3$, $R_2$, $R_3$, $R_4$, $R_5$ et $R_6$ ont les sens indiqués à la revendication 1,

$R_7$' représente un groupe hydrocarbyle en $C_1$ à $C_{16}$, substitué par un groupe pharmaceutiquement acceptable, spécifique d'une cible et capable de permettre à un complexe de métal, formé à partir de l'agent chélateur obtenu par réduction de la liaison disulfure du composé de formule (VI) de s'accumuler dans l'organe cible et provenant d'un composé choisi parmi une protéine ou une substance protéinique, un agent de fixation sur récepteur et une amine ; et

$R_8$' représente un atome d'hydrogène ou un groupe hydrocarbyle en $C_1$ à $C_{16}$, éventuellement substitué par un groupe pharmaceutiquement acceptable spécifique d'une cible.

**8.** Procédé de préparation d'un composé selon la revendication 7, caractérisé en ce qu'on fait réagir un précurseur de formule générale :

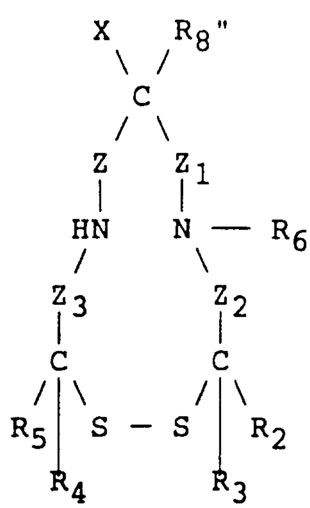

(VII)

dans laquelle

Z, $Z_1$, $Z_2$, $Z_3$, $R_2$, $R_3$, $R_4$, $R_5$ et $R_6$ ont les sens indiqués à la revendication 1,

X représente un groupe hydrocarbyle en $C_1$ à $C_{16}$, substitué par un groupe permettant d'obtenir par dérivation un groupe spécifique d'une cible, ce groupe permettant l'obtention d'un dérivé ou d'une dérivation étant choisi parmi un atome d'halogène (cet atome d'halogène étant choisi parmi un atome

de chlore, un atome de brome et un atome d'iode), un groupe aldéhyde, un carboxy et un groupe ester activé, et

$R_8$'' représente un atome d'hydrogène ou un groupe hydrocarbyle en $C_1$ à $C_{16}$, éventuellement substitué par un groupe permettant d'obtenir par dérivation un groupe spécifique d'une cible;

ou un sel de ce composé avec un acide minéral ou organique, avec un composé comprenant ledit groupe spécifique d'une cible.

**9.** Procédé selon la revendication 8, caractérisé en ce que, dans le précurseur de formule (VII), le groupe permettant de former un dérivé (ou permettant une dérivation) est un atome d'halogène choisi parmi un atome de chlore, un atome de brome et un atome d'iode.

**10.** Procédé selon la revendication 8 ou la revencidation 9, caractérisé en ce que le composé comprenant le groupe spécifique d'une cible est une amine, ou une protéine ou une substance protéinique.

**11.** Complexe d'un métal choisi parmi des radionucléides, des métaux du 7e et du 8e sous-groupe, et des lanthanides, avec un agent chélateur, selon l'une quelconque des revendications 3 à 6.

**12.** Procédé de préparation d'un complexe d'un métal, ce métal étant choisi parmi des radionucléides, des métaux des 7e et 8e sous-groupes, et des lanthanides, avec un agent chélateur, ledit complexe étant destiné à une application diagnostique ou thérapeutique, procédé caractérisé en ce qu'on soumet un sel ou un chélate dudit métal à une réaction de chélatation avec un composé selon l'une quelconque des revendications 3 à 6.

**13.** Composition radiothérapeutique comprenant, en plus d'un véhicule liquide pharmaceutiquement acceptable, un complexe d'un émetteur de rayons $\beta$ ou d'un autre radionucléide convenant pour une radiothérapie, composition caractérisée en ce qu'elle comprend un complexe selon la revendication 11 ou un complexe préparé selon un procédé selon la revendication 12.

**14.** Composition pour radiodiagnostic comprenant, en plus d'un véhicule liquide pharmaceutiquement acceptable, un complexe d'un radionucléide convenant pour un dosage de type radiologique, composition caractérisée en ce qu'elle comprend un complexe selon la revendication 11 ou un complexe préparé selon un procédé selon la revendication 12.

**15.** Ensemble ou nécessaire pour préparer une composition radiopharmaceutique, comprenant (i) en un état éventuellement sec une préparation d'un composé selon l'une quelconque des revendications 3 à 6, auquel est ou sont ajoutés, si on le désire, un support ou véhicule inerte pharmaceutiquement acceptable et/ou un ou plusieurs agents de formulation et/ou une ou plusieurs substances auxiliaires, (ii) une solution d'un sel ou d'un chélate d'un radionucléide métallique, et (iii), si on le désire, des instructions pour utilisation avec une prescription pour mettre (ii) en réaction de chélatation avec (i).

**16.** Ensemble ou nécessaire pour préparer une composition radiopharmaceutique, comprenant (i) en un état éventuellement sec une préparation d'un composé selon l'une quelconque des revendications 3 à 6, auquel, si on le désire, est ou sont ajoutés un excipient ou véhicule inerte pharmaceutiquement acceptable et/ou un ou plusieurs agents de formulation et/ou une ou plusieurs substances auxiliaires, (ii) un agent réducteur et; si on le désire, un agent chélateur, et (iii) si on le désire, des instructions pour l'utilisation avec une prescription de mettre du technétium-99m sous forme d'une solution de pertechnétate, ou du rhénium-186 ou du rhénium-188 sous forme d'une solution de perrhénate, en réaction de chélatation avec les ingrédients (i) et (ii).

**17.** Procédé pour soumettre un être vivant à sang chaud à un essai de dosage radiologique, caractérisé en ce qu'on administre à cet être une composition telle que revendiquée à la revendication 14, si on le désire après dilution avec un liquide pharmaceutiquement acceptable, la quantité de radioactivité administrée étant suffisante pour permettre une détection, à l'aide d'une formation d'image externe, et en ce qu'on soumet cet être à la formation d'une image externe pour déceler la radioactivité accumulée.

**18.** Procédé selon la revendication 17, dans lequel la quantité de radioactivité administrée est de 0,1 à 30 millicuries pour 70 kg de poids corporel.